Europäisches Patentamt

European Patent Office

Office européen des brevets

(19)

(11) Numéro de publication: **0 039 285**

**A1**

(12) **DEMANDE DE BREVET EUROPEEN**

(21) Numéro de dépôt: **81400644.1**

(22) Date de dépôt: **23.04.81**

(51) Int. Cl.³: **A 61 N 1/36**

(30) Priorité: **25.04.80 FR 8009363**

(43) Date de publication de la demande:
**04.11.81 Bulletin 81/44**

(84) Etats contractants désignés:
**BE CH DE GB IT LI LU NL SE**

(71) Demandeur: **CARDIOFRANCE- COMPAGNIE FRANCAISE D'ELECTROCARDIOLOGIE**
**Zone Industrielle Les Richardets 1 bis, rue des Aérostiers**
**F-93160 Noisy Le Grand(FR)**

(72) Inventeur: **Buffet, Jacques**
**28, avenue Thiers**
**F-93340 Le Raincy(FR)**

(74) Mandataire: **Derambure, Christian**
**BUGNION ASSOCIES SARL 116, boulevard Haussmann**
**F-75008 Paris(FR)**

(54) Procédé d'identification de l'état interne effectif d'un stimulateur cardiaque programmable implanté ainsi que le stimulateur permettant la mise en oeuvre du procédé.

(57) L'invention concerne un procédé d'identification de l'état interne effectif d'un stimulateur cardiaque programmable implanté et le stimulateur permettant la mise en oeuvre du procédé.

On commande le stimulateur pour qu'il fonctionne sous mode asynchrone et avant de lui donner toute instruction de programmation, on identifie que son fonctionnement correspond effectivement au mode aynchrone en constatant sur les moyens de visualisation du programmateur externe une succesion de cycles de spikes tel que les intervalles de temps successifs entre deux spikes voisins soient successivement $T - \Delta T$, $T + \Delta T$, $T + \Delta T$, $T - \Delta T$, ainsi de suite.

Croydon Printing Company Ltd.

EP 0 039 285 A1

Procédé d'identification de l'état interne effectif d'un stimulateur cardiaque programmable implanté ainsi que le stimulateur permettant la mise en oeuvre du procédé.

Les stimulateurs cardiaques implantables, programmables par voie externe sont connus. Ils comportent, outre les moyens de stimulation proprement dit, des moyens de réception d'un message, des moyens de décodage du message en une instruction ; des moyens de stockage de l'instruction correspondant au message réceptionné. A un tel stimulateur cardiaque est associé un programmateur externe comportant des moyens de commande permettant la réalisation d'un message correspondant à une instruction destinée au stimulateur et des moyens de visualisation des spikes du stimulateur.

Le programmateur peut faire l'objet de nombreuses formes d'exécution, selon le mode de transmission utilisée ; champ magnétique continu, tournant ou pulsé, ondes radio-électriques, ultra-sons, courant électrique. Le message correspondant à l'instruction à programmer est généralement transcrit sous la forme d'un mot en langage binaire. Les paramètres du stimulateur cardiaque qui sont programmables sont généralement les suivants : fréquence de stimulation, degré d'hystérésis, période réfractaire, largeur de l'impulsion, amplitude de l'impulsion sensibilité du stimulateur, mode de fonctionnement du stimulateur (sentinelle, synchrone, asynchrone).

Un stimulateur cardiaque implanté ayant été programmé c'est-à-dire une instruction de programmation lui ayant été envoyée par voie externe, il convient de s'assurer que son état effectif correspond à l'instruction donnée, c'est-à-dire que le stimulateur cardiaque a effectivement réceptionné l'instruction, l'a décodée correctement et l'a stockée pour l'utiliser.

A ce jour, ce contrôle est généralement réalisé a postériori en examinant le fonctionnement effectif du stimulateur selon le travail qui est fourni, une fois l'instruction de programmation donnée. Ce contrôle intervient donc un certain temps après que l'instruction de programmation ait été donnée. Ce contrôle intervient en outre seulement lorsque le stimulateur est en service effectif ce qui peut être plus ou moins long. Enfin, les informations de contrôle recueillies par voie externe sont toujours susceptibles d'être perturbées par des éléments exogènes. Ainsi donc, les procédés d'identification connus ne permettent pas de connaître avec certitude, précision et rapidité l'état interne effectif du stimulateur aussitôt après sa programmation et avant sa mise en service effective.

La présente invention vise donc à pallier ces inconvénients. Elle propose à cet effet un procédé d'identification de l'état interne effectif d'un stimulateur cardiaque programmable implanté dans lequel on commande d'abord le stimulateur pour qu'il fonctionne sous mode asynchrone et on identifie ensuite que le fonctionnement du stimulateur correspond effectivement au mode asynchrone en constatant sur les moyens de visualisation du programmateur que les intervalles de temps successifs, entre deux spikes voisins sont successivement $T-\Delta T$, $T+\Delta T$, $T+\Delta T$, $T-\Delta T$ et ainsi de suite. Après qu'une instruction ait été transmise au stimulateur cardiaque par le programmateur, on identifie l'état interne effectif de fonctionnement du stimulateur en analysant sur les moyens de visualisation du programmateur de la succession de $N = n_0 + n_1 + \ldots n_i + \ldots n_p$

cycles d'impulsions chacun de période 2T, comportant une impulsion intermédiaire se trouvant dans l'une des 2k positions possibles symétriques par rapport à la position médiane, la $m^{\text{ème}}$ position étant écartée de cette position médiane d'un côté ou de l'autre d'une valeur $m\Delta T$. On compare ces cycles avec un diagramme de référence.

Suivant l'invention, on affecte les premiers $n0$ cycles à l'identification du stimulateur contrôlé, les $n1$ cycles suivants à un premier paramètre ainsi défini, les $ni$ cycles à un ième paramètre et les derniers $np^{\text{ème}}$ cycle au $p^{\text{ème}}$ paramètre.

Le procédé suivant l'invention présente l'avantage de permettre de connaître l'état effectif, le fonctionnement du stimulateur immédiatement après qu'il ait été programmé et avant mêmeıqu'il n'entre en service effectif. Le procédé permet en effet d'identifier d'abord la nature du stimulateur cardiaque implanté puis la valeur de chacun des paramètres.

L'invention sera bien comprise grâce à la description suivante.

L'invention concerne un procédé d'identification de l'état interne effectif d'un stimulateur cardiaque programmable et implanté.

Ce stimulateur cardiaque comporte des moyens de stimulation, des moyens de réception d'un message en provenance d'un programmateur externe, des moyensde décodage de ce message en une instruction de programmation et des moyens de stockage de cette instruction.

Le programmateur externe comporte des moyens d'affichage d'une instruction (tel qu'un clavier) et des moyens de visualisation des impulsions de stimulation. Ce programmateur qui ne fait pas directement partie de l'invention peut faire l'objet de nombreuses formes d'exécution selon le mode de transmission employée (champ magnétique continu, tournant ou pulsé, ondes radio-électriques, ultra-sons, courant électrique). L'association du programmateur au stimulateur cardiaque, par l'intermédiaire du patient, peut également faire l'objet de nombreuses formes d'éxécution (par exemple le programmateur comporte deux bracelets fixés aux poignets du patient). L'opérateur affiche sur le programmateur un message sous la forme d'un mot en langage binaire, correspondant à une instruction de programmation.

Le stimulateur cardiaque peut comporter plusieurs paramètres programmables, non limitativement.

Dans une forme d'exécution classique, le stimulateur cardiaque comporte six paramètres programmables ; fréquence de stimulation, hystérésis, période réfractaire, largeur d'impulsion, amplitude d'impulsion, sensibilité. En outre, le stimulateur cardiaque comporte un paramètre qui est son mode de fonctionnement. Il comporte enfin un paramètre qui est son seuil de fonctionnement. Ainsi, le fonctionnement d'un tel stimulateur cardiaque est bien défini par ces huit paramètres précédemment mentionnés.

Chacun des paramètres varie de façon discrète et peut prendre une valeur parmi plusieurs valeurs fixées.

Ainsi, la fréquence de stimulation peut correspondre aux seize valeurs de périodes suivantes (exprimées millisecondes) ; 500, 550, 600, 650, 700, 750, 800, 850, 900, 950, 1000, 1050, 1100, 1150, 1200 et 1250. L'hystérésis est également susceptible de prendre une valeur parmi les seize suivantes (exprimées en milli secondes) : 500, 550, 600, 650, 700, 750, 800, 850, 900, 950, 1000, 1050, 1100, 1150, 1200, 1250. La période réfractaire est susceptible de prendre une valeur parmi les huit valeurs suivantes (exprimées
en millisecondes) 100, 150, 200, 250, 300, 350, 400 et
450. La largeur d'impulsion est susceptible de prendre
une valeur parmi les quinze valeurs suivantes (exprimées
en millisecondes) 0,1 , 0,2 , 0,3 , 0,4 , 0,5 , 0,6 ,
0,7, 0,8 , 0,9 , 1 , 1,1, 1,2 , 1,3 , 1,4 et 1,5 (cette
dernière valeur n'étant obtenue que sous aimant). L'amplitude peut prendre une valeur parmi les trois valeurs suivantes (exprimées en volts) ; 2,5 - 5 et 7,5 (cette dernière
valeur n'étant obtenue que sous aimant). La sensibilité
peut prendre l'une des valeurs parmi les deux suivantes
(exprimées en millivolts) : 0,9 et 1,8. Le mode de fonctionnement est l'un des trois modes suivants : sentinelle,
synchrone, asynchrone de période T. Le seuil peut prendre
l'une des trois valeurs suivantes (exprimées en volts) :
2,5 - 5 et 7,5.

Naturellement, l'invention s'applique à un nombre de paramètres programmables différents, chacun pouvant prendre
un nombre de valeurs différentes.

L'invention sera présentée dans le cadre général d'un paramètre ayant p paramètre programmable.

Dans le procédé suivant l'invention, on commande d'abord
le stimulateur cardiaque pour qu'il fonctionne suivant le
mode asynchrone. A cet effet, on place par exemple le
stimulateur cardiaque sous aimant.

Ensuite et avant de donner au stimulateur cardiaque toute
instruction de programmation , on identifie que le fonctionnement du stimulateur cardiaque correspond effectivement au
mode asynchrone. A cet effet, l'opérateur observe les moyens de visualisation du programmateur et constate sur ceux-
ci une succesion de cycles tel que les intervalles de temps
successifs entre deux impulsions voisines sont successivement

les suivants : $T - \Delta T$, $T + \Delta T$, $T + \Delta T$, $T - \Delta T$ et ainsi de suite. Autrement dit, l'opérateur constate l'existence de cycles d'impulsions de période 2T comportant une impulsion intermédiaire qui bascule successivement de part et d'autre du point médian du cycle, d'une valeur $\Delta T$. Naturellement, il est clair que le stimulateur cardiaque est programmé pour que son fonctionnement sous mode asynchrone correspond à l'émission d'impulsions selon les cycles indiqués.

Ensuite, le procédé vise à identifier pour chaque paramètre la valeur correspondant à l'état effectif dans lequel doit fonctionner le stimulateur, valeur qui est choisie parmi les différentes valeurs susceptibles d'être sélectionnéés . Egalement, et préférentiellement, le procédé vise à permettre de déterminer la nature ou catégorie même du stimulateur cardiaque.

A cet effet, après que le stimulateur ait été placé en mode de fonctionnement asynchrone et que l'opérateur ait contrôlé que le stimulateur fonctionne effectivement sur ce mode, l'opérateur peut grâce au programmateur donner au stimulateur une ou plusieurs instructions selon les procédés de programmation connus en soi.

Après que ces instructions aient été données, l'opérateur identifie l'état interne effectif de fonctionnement du stimulateur, conformément au procédé suivant l'invention. A cet effet, l'opérateur analyse les spikes successifs apparaissant sur les moyens de visualisation du programmateur. Plus précisément, l'opérateur analyse $N = n_0 + N_1 + \ldots n_i + \ldots n_p$ cycle de spikes. Chaque cycle a une période 2T et comporte une impulsion intermédiaire se trouvant dans l'une de deux k positions possibles symétriques par rapport au point médian du cycle. Deux positions voisines pour l'impulsion intermédiaire sont écartées de $\Delta T$. Les deux positions les plus voisines du point médian du cycle sont écartées

de celui-ci de T, d'un côté ou de l'autre. La m$^{ème}$ position de l'impulsion intermédiaire est écartée du point
médian d'un côté ou de l'autre d'une valeur m $\Delta$ T,
m étant un nombre entier compris entre 1 et k.

Par convention, chacun des groupes de cycles (n0 , ... ni
... np) est affecté à un élément à identifier à savoir le
type de stimulateur et les différents paramètres programmables.

Par exemple, les n0 cycles sont affectés à l'identification
même du stimulateur cardiaque. Les n1 cycles sont affectés
à un premier paramètre ainsi défini, les ni$^{ème}$ cycles à un
i$^{ème}$ paramètre ainsi défini et les np$^{ème}$ cycle au p$^{ème}$
paramètre.

Pour chaque groupe de cycles, soit par exemple les ni$^{ème}$
cycles correspondant au i$^{ème}$ paramètre, est affecté, par
convention, une valeur déterminée dudit paramètre à chaque
position relative des impulsions intermédiaires. Naturellement, le stimulateur cardiaque est conçu pour qu'en réponse
à une instruction sur la valeur d'un paramètre, il émette
des impulsions successives correspondant aux positions
convenues des impulsions intermédiaires. L'opérateur peut
donc disposer de tableaux, graphiques, plus généralement
images de référence, donnant pour chaque groupe de cycles,
soit par exemple les ni$^{ème}$ cycles les différentes valeurs
correspondant aux différentes positions des impulsions intermédiaires. L'opérateur peut donc comparer les cycles
apparaissant sur les moyens de visualisation (cycles éventuellement imprimés simultanément) aux images qu'il possède et déterminer pour chaque paramètre quelle est sa valeur
effective.

En pratique l'opérateur examine d'abord quel est le type
de stimulateur par les n0 premiers cycles puis successivement les p paramètres programmables grâce aux n1, ... ni ... np cycles.

Dans un cas typique, le nombre de paramètres programmables est égal à 8, donc p = 8. Le nombre de cycles nécessaires pour chaque paramètre dépend du nombre de valeurs possibles pour ce paramètre et du nombre des positions possibles pour l'impulsion intermédiaire. Dans une forme d'exécution possible, k = 4. Alors, chaque paramètre qui peut prendre au plus huit valeurs nécessite seulement un cycle. En l'occurrence, les paramètres suivants : fréquence de stimulation, hystérésis, largeur d'impulsions nécessitent deux cycles et les paramètres suivants ; période réfractaire, amplitude, sensibilité, mode de fonctionnement et seuil un cycle seulement.

En ce qui concerne la nature même du stimulateur, on peut considérer que n0 = 2 est suffisant pour permettre l'identification d'un grand nombre possible de types différents de stimulateurs.

Comme on le comprend bien, à chaque état possible du stimulateur correspond une et une seule succession déterminée d'impulsions des N cycles, susceptible d'être observée par l'opérateur pour pouvoir être comparée à des images de référence qui chacune pour une succession déterminée d'impulsions indiquent l'état correspondant du stimulateur.

Il est à noter que l'image observée par l'opérateur est appréciée selon la position relative de l'impulsion intermédiaire entre l'impulsion initiale et l'impulsion terminale du cyle. L'opérateur n'a donc pas à se préoccuper de la valeur absolue de la période du cycle. Il s'ensuit une sécurité plus importante.

L'invention concerne également le stimulateur cardiaque permettant la mise en oeuvre du procédé. Ce stimulateur cardiaque est initialement programmé pour pouvoir émettre des impulsions correspondant aux images ainsi expliquées précédemment.

Dans un mode d'utilisation classique, l'opérateur place le stimulateur en mode de fonctionnement asynchrone, puis vérifie que le fonctionnement est bien celui-ci. Ensuite, il peut programmer un ou plusieurs paramètres, grâce au programmateur et enfin contrôler, identifier l'état effectif de fonctionnement du stimulateur enobservant, analysant et comparant les N cycles d'impulsions successifs ce qui lui permet de considérer successivement l'identification même du stimulateur puis chacun des paramètres possibles.

Naturellement, l'invention peut faire l'objet de nombreuses variantes d'exécution.

Revendications de brevet.

1. Procédé d'identification de l'état interne effectif d'un stimulateur cardiaque programmable implanté, caractérisé par le fait qu'on commande le stimulateur pour qu'il fonctionne sous mode asynchrone et avant de lui donner toute instruction de programmation, on identifie que son fonctionnement correspond effectivement au mode asynchrone en constatant sur les moyens de visualisation du programmateur externe une succession de cycles d'impulsions tel que les intervalles de temps successifs entre deux spikes voisins soient successivement $T - \Delta T$, $T + \Delta T$, $T + \Delta T$, $T - \Delta T$ et ainsi de suite.

2. Procédé suivant la revendication 1, caractérisé par le fait qu'après avoir transmis au stimulateur cardiaque au moins une instruction correspondant à la valeur d'un paramètre, on identifie l'état interne effectif du stimulateur en analysant sur les moyens de visualisation du programmateur externe une succesion de $N = n0 = n1 + ... ni + ... np$ cycles d'impulsions, chacun de période 2T comportant une impulsion intermédiaire se trouvant dans l'une des $\Delta K$ positions possibles symétriques par rapport à la position médiane, la $m^{ème}$ position étant écartée de cette position médiane, d'un côté ou de l'autre, d'une valeur $m \Delta T$, chaque état possible du stimulateur cardiaque correspondant à une et une seule succession déterminée d'impulsions et on compare les N cycles d'impulsions avec des images de référence correspondant aux différents états possibles du stimulateur cardiaque.

3. Procédé suivant l'une quelconque des revendications 1 et 2, caractérisé par le fait qu'on affecte les n0 cycles à l'identification du type même de stimulateur, les $n_i$ cycles à un $i^{ème}$ paramètre.

4. Procédé selon l'une quelconque des revendications 1 à 3, caractérisé par le fait que k = 4, p = 8, n0, n1, .... ni ..., np étant égaux à un ou deux.

5. Stimulateur cardiaque programmable et implantable, caractérisé par le fait qu'il permet le procédé selon l'une quelconque des revendications 1 à 4, à savoir qu'il est programmé initialement pour pouvoir émettre des impulsions correspondant à des images de référence, pour chaque état possible.

## Office européen des brevets

# RAPPORT DE RECHERCHE EUROPEENNE

**0039285**
Numéro de la demande

EP 81 40 0644

| DOCUMENTS CONSIDERES COMME PERTINENTS | | | CLASSEMENT DE LA DEMANDE (Int. Cl.³) |
|---|---|---|---|
| Catégorie | Citation du document avec indication, en cas de besoin, des parties pertinentes | Revendication concernée | |
| | US - A - 4 190 055 (ARCO)<br>* Colonne 1, lignes 36-56; colonne 3, ligne 60 à colonne 4, ligne 17; colonne 5, lignes 28-59; colonne 6, lignes 1-6 * | 1-5 | A 61 N 1/36 |
| | FR - A - 2 400 888 (MEDCOR)<br>* Page 5, lignes 6-34 * | 1 | |
| | US - A - 3 026 504 (RCA)<br>* Colonne 3, ligne 63 à colonne 4, ligne 10; colonne 4, lignes 40-67 * | 2 | **DOMAINES TECHNIQUES RECHERCHES (Int. Cl.³)**<br><br>A 61 N 1/36<br>1/08<br>G 08 C 19/24 |
| | FR - A - 2 379 104 (PACESETTER)<br>* Page 5, lignes 8-24; page 6, lignes 19-35; page 17, lignes 7-22; page 20, lignes 1-12 * | 2,3 | |
| | US - A - 3 257 651 (FEISEL)<br>* Colonne 1, lignes 53-57; colonne 4, lignes 54-66 * | 2 | **CATEGORIE DES DOCUMENTS CITES** |
| | IEEE TRANSACTIONS ON BIOMEDICAL ENGINEERING, vol. BME-21, no. 4, juillet 1974<br>New York, US<br>W.C. LIN et al. "A micropower pulse-width-modulation-pulse-position-modulation two-channel telemetry system for biomedical | 2 | X: particulièrement pertinent<br>A: arrière-plan technologique<br>O: divulgation non-écrite<br>P: document intercalaire<br>T: théorie ou principe à la base de l'invention<br>E: demande faisant interférence<br>D: document cité dans la demande<br>L: document cité pour d'autres raisons |
| | | ./. | &: membre de la même famille, document correspondant |

Le présent rapport de recherche a été établi pour toutes les revendications

| Lieu de la recherche | Date d'achèvement de la recherche | Examinateur |
|---|---|---|
| La Haye | 31-07-1981 | SIMON |

OEB Form 1503.1 06.78

**0039285**
Numéro de la demande

**RAPPORT DE RECHERCHE EUROPEENNE**

Office européen
des brevets

EP 81 40 0644
-2-

| Catégorie | Citation du document avec indication, en cas de besoin, des parties pertinentes | Revendication concernée | CLASSEMENT DE LA DEMANDE (Int. Cl. ³) |
|---|---|---|---|
| | **DOCUMENTS CONSIDERES COMME PERTINENTS** | | |
| | applications", pages 273-280. <br> * Page 274, alinéas 4-6; page 275, figure 3 (mode 1) * <br><br> -- | | |
| EP | EP - A - 0 011 936 (MEDTRONIC) <br> * Page 4, dernier paragraphe; page 5, paragraphe 1; 'claims' 1-5,8 * <br> & DE - A - 2 944 635 <br> -- | 1,2,5 | |
| EP | EP - A - 0 011 935 (MEDTRONIC) <br> * Page 57, lignes 3-23;"claims" 5,6 * <br> -- | 2,5 | **DOMAINES TECHNIQUES RECHERCHES (Int. Cl. ³)** |
| A | US - A - 4 088 139 (MEDALERT) | 1,2 | |
| A | FR - A - 2 368 943 (MEDTRONIC) <br><br> ----- | 1,2 | |

OEB Form 1503.2 06.78